# EUROPÄISCHE PATENTSCHRIFT

(11) **EP 1 939 168 B1**
(45) Veröffentlichungstag und Bekanntmachung des Hinweises auf die Patenterteilung: **30.09.2009**
(21) Anmeldenummer: 07119244.7
(22) Anmeldetag: 25.10.2007
(51) Int. Cl.: C07C 209/68, C07C 211/09

(54) **Kontinuierliches Verfahren zur Decarboxylierung von Carbonsäuren**
Continuous method for decarboxylation of carboxylic acid
Procédé continu destiné à la décarboxylation d'acides carboniques

(30) Priorität: 20.12.2006 DE 102006060908
(43) Veröffentlichungstag der Anmeldung: 02.07.2008
(73) Patentinhaber: Evonik Degussa GmbH, 45128 Essen (DE)
(72) Erfinder: Omeis, Marianne, 46286 Dorsten (DE); Köhler, Günther, 45770 Marl (DE); Neumann, Manfred, 45770 Marl (DE); Kübelbäck, Thomas, 48249 Dülmen (DE)

(56) Entgegenhaltungen:
- EP-A- 1 586 553
- WO-A-00/39098
- JP-A- 2004 000 114

## Beschreibung

Die Erfindung betrifft ein Verfahren zur Decarboxylierung von Carbonsäuren.

Die Decarboxylierung von Aminosäuren ist ein gebräuchliches Verfahren um Aminoverbindungen herzustellen. Die erhaltenen Aminoverbindungen sind wiederum Ausgangsverbindungen für zahlreiche technische Zwischenprodukte und Pharmawirkstoffe.

Den Reaktionsmechanismus für eine Decarboxylierung von Aminosäuren in Gegenwart von Ketonen als Katalysator beschreibt Chatelus in Bull. Soc. Chim. Fr., 1964, 2523 - 2532:

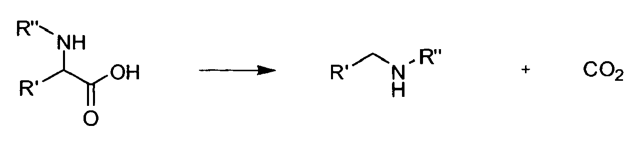

Hashimoto et al. beschreiben in Chemistry Letters (1986) 893 - 896 die Decarboxylierung von α-Aminosäuren unter Einsatz von 2-Cyclohexen-1-on als Katalysator.

Auch die JP 4010452 B beschreibt ein Verfahren zur Decarboxylierung von Aminosäuren, wobei die Aminosäure in dem Lösemittel Cyclohexanol vorgelegt wird und der Katalysator 2-Cyclohexen-1-on zugegeben wird.

Die in dem Stand der Technik beschriebenen Verfahren setzen Aminosäuren als Reinsubstanz ein. Im Handel sind jedoch diese Aminosäuren häufig nur als wässrige Lösungen erhältlich. Die Gewinnung der Reinsubstanz aus der wässrigen Lösung kann sich im Einzelfall äußerst schwierig und aufwändig gestalten. Ferner handelt es sich bei den Verfahren gemäß dem Stand der Technik um diskontinuierliche Verfahren.

Es war daher Aufgabe der vorliegenden Erfindung, ein Verfahren zur Decarboxylierung von Carbonsäuren zur Verfügung zu stellen, das es ermöglicht die Carbonsäuren - insbesondere die Aminosäuren - als wässrige Lösung einzusetzen. Insbesondere war es die Aufgabe ein kontinuierliches Verfahren zur Verfügung zu stellen.

Überraschenderweise wurde gefunden, dass die zu decarboxylierende Carbonsäure als wässrige Lösung bei dem erfindungsgemäßen Verfahren eingesetzt werden kann, wenn die Carbonylverbindung, die als Katalysator dient, in einem Lösemittel vorgelegt wird und die wässrige Lösung der zu decarboxylierenden Carbonsäure zudosiert wird. Der Mechanismus der Decarboxylierung wird gemäß dem Stand der Technik über die Schiffsche Base-Reaktion erklärt, Umso mehr war es überraschend, dass es möglich ist, die zu decarboxylierende Carbonsäure als wässrige Lösung einzusetzen, da Schiffsche Base-Reaktionen bekanntlich unter Wasserabspaltung verlaufen, um hierbei einen Reaktionsfortschritt zu erzielen. Mittels dem erfindungsgemäßen Verfahren ist es nun möglich, handelsübliche Aminosäuren, die vorwiegend als wässrige Lösung am Markt verfügbar sind, direkt für die Decarboxylierung einzusetzen, ohne dass vorher in einem aufwändigen Verfahren die wasserfreie Carbonsäure - insbesondere Aminosäure - aus der wässrigen Lösung gewonnen werden muss. Bei den entsprechenden Reaktionstemperaturen, bei der i.d.R. solch eine Decarboxylierung stattfindet, kann das Decarboxylierungsprodukt zusammen mit dem CO₂, Lösemittel und dem Wasser als Dampf aus dem Reaktionsgemisch entfernt werden. Auf diese Weise ist es nun möglich, ein kontinuierliches Verfahren zur Verfügung zu stellen. Das gewünschte Decarboxylierungsprodukt kann dampfförmig aus der Reaktionszone entfernt und als Gemisch - beispielsweise mittels Wasserabscheiders - einfach abgetrennt werden. Das Lösemittel bildet i.d.R. die organische Phase, während die wässrige Phase das gewünschte Produkt bzw. eine Vorstufe des gewünschten Produkts aufweist. Die wässrige Phase kann nun beispielsweise über ein thermisches Trennverfahren aufgearbeitet werden.

Gegenstand der Erfindung ist ein kontinuierliches Verfahren zur Decarboxylierung von Carbonsäuren gemäß der Formel I, 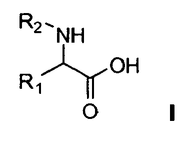
mit R¹ = Wasserstoff, Alkyl-, Arylalkyl-, Aryl-, Cycloalkyl- oder Heterocyclus, wobei der Substituent vom Typ R₁ substituiert oder unsubstituiert ist, und
R² = Wasserstoff, Alkyl, Cycloalkyl
das sich dadurch auszeichnet, dass
I. eine Carbonylverbindung als Katalysator in einem Lösemittel bei Reaktionstemperatur vorgelegt wird,
II. eine Carbonsäure gemäß der Formel I als wässrige Lösung, wässrige Suspension oder als wasseraufweisender Feststoff dem Katalysator in der Reaktionszone zudosiert wird und
III. ein Gemisch aus CO₂, Lösemittel, Wasser und dem gewünschten Reaktionsprodukt gemäß Formel II und / oder einem Salz, welches ein Anion gemäß der Formel III aufweist, 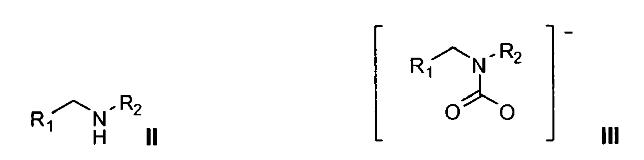 als Dampf aus dem Reaktionsgemisch kontinuierlich entfernt wird.

Als Carbonsäuren werden in dem erfindungsgemäßen Verfahren Verbindungen gemäß der Formel I eingesetzt: 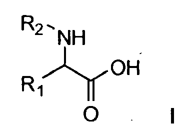 mit R₁ = Wasserstoff, Alkyl-, Arylalkyl-, Aryl-, Cycloalkyl- oder Heterocyclus, wobei der Substituent vom Typ R₁ substituiert oder unsubstituiert ist, und R₂ = Wasserstoff, Alkyl, Cycloalkyl. Als weitere Substituenten am Substituenten vom Typ R₁ kann dieser die folgenden funktionellen Gruppen aufweisen:
- OH, -COOH, -CONH₂, -SH, -S-Alkyl, -NH₂, -NH-CH(NH₂)₂, -S-S-CH₂-CH(NH₂)-COOH,
- NH-CO-NH₂, -CO-NH-Alkyl, -S⁺(Alkyl)₂, -SO-CH₂-CH=CH₂, -O-PO(OH)₂.

Insbesondere werden in dem erfindungsgemäßen Verfahren natürlich vorkommende Aminosäuren eingesetzt.

Die Substituenten vom Typ R₁ und R₂ können in einer besonderen Ausführungsform des erfindungsgemäßen Verfahrens ein Ringssystem ausbilden, besonders bevorzugt werden hierbei die natürlich vorkommenden Aminosäure Prolin bzw. Derivate des Prolins eingesetzt.

Vorzugsweise werden in dem erfindungsgemäßen Verfahren α-Aminosäuren gemäß der Formel IV eingesetzt.

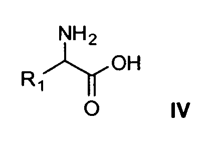

Bevorzugt werden in dem erfindungsgemäßen Verfahren jedoch Verbindungen gemäß der Formel V eingesetzt: 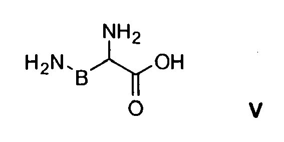 mit B = -Alkyl-, -Arylalkyl-, -Aryl-, -Cycloalkyl- oder -Heterocyclus-. Besonders bevorzugt werden in dem erfindungsgemäßen Verfahren jedoch Carbonsäuren der Formel VI 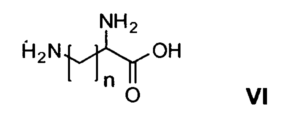 mit n = 1 bis 5 eingesetzt. Ganz besonders bevorzugt werden in dem erfindungsgemäßen Verfahren α-Aminosäuren, ausgewählt aus Lysin und Ornithin, eingesetzt.

Die zu decarboxylierenden Carbonsäuren können in dem erfindungsgemäßen Verfahren als wässrige Lösung, wässrige Suspension oder als wasseraufweisender Feststoff eingesetzt werden.

In dem erfindungsgemäßen Verfahren wird vorzugsweise für den Katalysator ein Lösemittel mit einem Siedepunkt von 150°C bis 390°C, besonders bevorzugt von 180°C bis 220°C eingesetzt. Das Lösemittel für den Katalysator weist in dem erfindungsgemäßen Verfahren vorzugsweise eine wasserschleppende Wirkung auf, dies bedeutet im Sinne dieser Erfindung, dass das Lösemittel ein Schleppmittel für Wasser ist. Besonders bevorzugt wird ein Lösemittel, ausgewählt aus 2-Ethylhexanol, Dibenzyltoluol und Isononanol, in dem erfindungsgemäßen Verfahren eingesetzt. Eine Mischung aus mehreren geeigneten Lösemitteln kann in dem erfindungsgemäßen Verfahren ebenfalls als Lösemittel eingesetzt werden.

Durch den Einsatz von Lösemittel mit einem hohen Siedepunkt ist eine hohe Reaktionstemperatur bei der Zudosierung der wässrigen Lösung der Carbonsäure während der Decarboxylierung möglich. Hierdurch kann das den Reaktionsmechanismus der Decarboxylierung störende Wasser, welches durch die wässrige Darreichungsform der Carbonsäure in das Reaktionsgemisch bzw. in die Reaktionszone eingetragen wird, rasch wieder aus der Reaktionszone des Reaktors entfernt werden. Insbesondere erfolgt der Austrag des Wassers sehr gut, wenn das in dem erfindungsgemäßen Verfahren eingesetzte Lösemittel auch eine wasserschleppende Wirkung aufweist.

Die Decarboxylierung des erfindungsgemäßen Verfahrens wird vorzugsweise bei einer Reaktionstemperatur von 140°C bis 240 °C, bevorzugt von 170°C bis 210°C durchgeführt.

Der Druck, bei dem die Decarboxylierung des erfindungsgemäßen Verfahrens durchgeführt wird, beträgt vorzugsweise von 20 mbar bis 2000 mbar, bevorzugt von 800 bis 1200 und besonders bevorzugt von 950 bis 1100. Ganz besonders bevorzugt wird die Decarboxylierung des erfindungsgemäßen Verfahrens bei Atmosphärendruck durchgeführt.

Als Katalysator werden bei der Decarboxylierung des erfindungsgemäßen Verfahrens vorzugsweise hochsiedende Carbonylverbindungen, insbesondere cyclische oder acyclische Ketone oder Aldehyde, besonders bevorzugt wird jedoch 2-Cyclohexen-1-on oder Isophoron eingesetzt. Unter hochsiedenden Carbonylverbindungen werden Carbonylverbindungen mit einem Siedepunkt von größer 150°C im Rahmen dieser Erfindung verstanden.

Vorzugsweise werden in dem erfindungsgemäßen Verfahren von 0,005 bis 10 Moleq., bevorzugt von 0,007 bis 1 Moleq., besonders bevorzugt von 0,008 bis 0,05 Moleq. und ganz besonders bevorzugt von 0,009 bis 0,03 Moleq. an Katalysator bezogen auf die eingesetzte Menge an Carbonsäure eingesetzt.

In einer weiteren Ausführungsform werden in dem erfindungsgemäßen Verfahren von 0,09 bis 4,5 Moleq., bevorzugt 0,1 bis 1,5 Moleq. an Katalysator bezogen auf die eingesetzte Menge an Carbonsäure eingesetzt.

Aufgrund der starken CO₂-Entwicklung während der Decarboxylierung des erfindungsgemäßen Verfahrens ist es vorteilhaft einen Entschäumer dem Reaktionsgemisch zuzugeben. Hierbei können dem Fachmann geläufige Entschäumer, die sich gegenüber der Decarboxylierungsreaktion inert verhalten, eingesetzt werden. Vorzugsweise werden Silikonöle eingesetzt. Beispielsweise können Entschäumer eingesetzt werden, wie sie unter dem Handelsnamen Silikonöl AK 350 oder EXTRAN^{®} AP 81 vertrieben werden.

Vorzugsweise wird zunächst der Katalysator zusammen mit dem Lösemittel der Reaktionszone des Reaktors zugeführt. Anschließend wird vorzugsweise die Katalysatorlösung auf die gewünschte Reaktionstemperatur erwärmt bzw. erhitzt. Erst bei erreichen der gewünschten Reaktionstemperatur sollte die Zugabe der wässrigen Darreichungsform der Carbonsäure erfolgen. Um ein unkontrolliertes Aufschäumen des Reaktionsgemisches durch die starke CO₂-Entwicklung zu verhindern, wird vorteilhafterweise ein Entschäumer bereits vor Chargierung der Carbonsäure zur Katalysatorlösung zugegeben.

Das bei der Decarboxylierung freiwerdende Kohlendioxid, das in äquimolaren Mengen entsteht, dient als Strippgas für das Decarboxylierungsprodukt gemäß den Formeln II und/oder dem Salz, welches ein Anion gemäß der Formel III aufweist. Somit ist es möglich, dass dieses Decarboxylierungsprodukt aus der Reaktionszone abgezogen werden kann.

CO₂, Lösemittel, Wasser und das Decarboxylierungsprodukt werden dampfförmig aus der Reaktionszone entfernt. Dieses Produktgemisch kann nun in seine beiden Phasen - beispielsweise in einem Wasserabscheider - aufgetrennt werden. Die organische Phase, die in der Regel das Lösemittel darstellt, kann - insbesondere ohne weitere Aufarbeitung - dem Reaktor zurückgeführt werden. Die wässrige Phase enthält das Decarboxylierungsprodukt gemäß der Formel II und / oder einem Salz, welches ein Anion gemäß der Formel III aufweist. Dieses Salz wird in der Dampfphase aus dem Decarboxylierungsprodukt gemäß der Formel II und CO₂ gebildet. In der Regel liegt nach der Kondensation ein Gemisch aus dem Decarboxylierungsprodukt gemäß der Formel II und dem Salz, welches ein Anion gemäß der Formel III aufweist, vor. Die Aufarbeitung der wässrigen Phase erfolgt vorzugsweise über ein thermisches Verfahren, wobei hier die Abtrennung des Wassers als auch die Spaltung des Decarboxylierungsproduktes des Salzes, welches ein Anion gemäß der Formel III aufweist, in das Decarboxylierungsprodukts gemäß der Formel II und CO₂ erfolgen kann. Das thermische Verfahren wird vorzugsweise bei Inertgas durchgeführt.

Bevorzugt erfolgt die Aufarbeitung der wässrigen Phase in einem Verdampfer - beispielsweise in einem Fallfilm-, Dünnschicht oder Rieselfilmverdampfer - , wobei das Wasser verdampft wird und das Decarboxylierungsprodukt gemäß der Formel II im Sumpf des Verdampfers bereits in hoher Reinheit - insbesondere einer GC-Reinheit > 99,0 Flächen-% - anfällt. Unter den Bedingungen des Verdampfers können die Anteile des Decarboxylierungsproduktes, das als Salz bzw. als Carbamat vorliegen, wieder thermisch in das gewünschte Decarboxylierungsprodukt gemäß der Formel II und CO₂ gespalten werden. Es ist ratsam bei diesem Aufarbeitungsschritt unter Inertgas zu arbeiten, um die erneute Bildung von Carbamaten gemäß der Formel III, die sich aus dem Reaktionsprodukt und CO₂ aus der Atmosphäre bilden können, zu verhindern.

Sollte das Decarboxylierungsprodukt nach dem thermischen Verfahren nicht in der gewünschten Reinheit vorliegen, so kann in dem erfindungsgemäßen Verfahren eine weitere Destillation oder Rektifikation angeschlossen werden, um ein hochreines Produkt zu gewinnen.

Dieser weitere Destillations- bzw. Rektifikationsschritt sollte ebenfalls unter Inertgasatmosphäre, wie beispielsweise Stickstoff erfolgen.

Die nachfolgenden Beispiele sollen das erfindungsgemäße Verfahren zur Decarboxylierung von Carbonsäuren näher erläutern, ohne dass die Erfindung auf diese Ausführungsform beschränkt sein soll.

### Beispiel 1

400,0 g Dibenzyltoluol, 8,0 g Isophoron (0,06 mol) und 4,0 g Entschäumer (Silikonöl AK 350 der Fa. Wacker) werden in einem 2 l doppelwandigem, zylindrischem Reaktionsgefäß mit einem Innenthermometer, Flügelrührer mit einer Rührhülse und einem Rührmotor, einer 1 l Telabpumpe zur Dosierung der wässrigen Carbonsäurelösung, einer 10 cm langen verspiegelten Kolonne, die mit Raschigringen gefüllt ist, einem Wasserabscheider, einem Aufsatz für die Kopftemperatur und einem Kühler vorgelegt und unter Rühren erhitzt, wobei die Ölvorlauftemperatur am Thermostaten 240 °C beträgt. Insgesamt werden 1195 g einer 50 Gew.-% igen wässrigen Lysinlösung (entspricht 4,09 mol L-Lysin) mit einer Dosierrate von 199 g/h mittels einer Pumpe in den Sumpf eindosiert.

Wasser und das Decarboxylierungsprodukt Cadaverin fallen kontinuierlich als wässrige Phase am Wasserabscheider an, während Dibenzyltoluol als organische Phase im Wasserabscheider anfällt. Die wässrige Phase (783,5 g) wird abgetrennt, wobei der Cadaveringehalt dieser wässrigen Phase 14 Flächen-% beträgt (bestimmt mittels GC, Säule HP 5 und Detektor WLD). Die Aufarbeitung der wässrigen Phase erfolgt über einen Dünnschichtverdampfer (Höhe 44 cm, Wischerlänge 38 cm, Durchmesser 5,1 cm, Fläche des Dünnschichtverdampfers: 0,061 m², Inertgasspülung) bei einer Ölvorlauftemperatur von 185 °C und einer Dosierrate von 600 ml/h. Im Sumpf des Dünnschichtverdampfers fallen 97,8 g Cadaverin in einer GC-Reinheit von >97,0 Flächen-% (Detektor WLD) an. Die Ausbeute an Cadaverin beträgt bezogen auf das eingesetzte L-Lysin 23,4 %.

## Patentansprüche

1. Kontinuierliches Verfahren zur Decarboxylierung von Carbonsäuren gemäß der Formel I 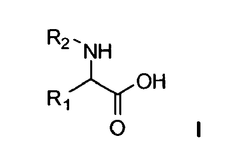 mit R¹ = Wasserstoff, Alkyl-, Arylalkyl-, Aryl-, Cycloalkyl- oder Heterocyclus, wobei der Substituent vom Typ R₁ substituiert oder unsubstituiert ist, und
R₂ = Wasserstoff, Alkyl, Cycloalkyl,
**dadurch gekennzeichnet,**
**dass**
I. eine Carbonylverbindung als Katalysator in einem Lösemittel bei Reaktionstemperatur vorgelegt wird,
II. eine Carbonsäure gemäß der Formel I als wässrige Lösung, wässrige Suspension oder als wasseraufweisender Feststoff dieser Katalysatorlösung zudosiert wird und
III. ein Gemisch aus CO₂, Lösemittel, Wasser und dem gewünschten Reaktionsprodukt gemäß Formel II und / oder einem Salz, welches ein Anion gemäß der Formel III aufweist, 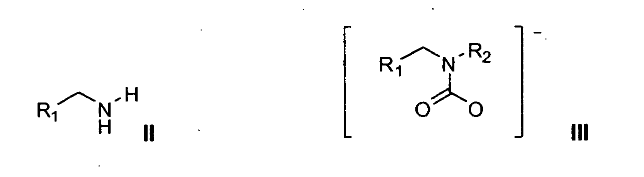 als Dampf aus dem Reaktionsgemisch kontinuierlich entfernt wird.

2. Verfahren gemäß Anspruch 1,
**dadurch gekennzeichnet,**
**dass** das Lösemittel eine wasserschleppende Wirkung aufweist.

3. Verfahren gemäß Anspruch 1 oder 2,
**dadurch gekennzeichnet,**
**dass** das Lösemittel einen Siedepunkt von 150°C bis 390°C aufweist.

4. Verfahren gemäß zumindest einem der Ansprüche 1 bis 3,
**dadurch gekennzeichnet,**
**dass** von 0,005 bis 10 Moleq. an Katalysator bezogen auf die eingesetzte Menge an Carbonsäure eingesetzt wird.

5. Verfahren gemäß Anspruch 4,
**dadurch gekennzeichnet,**
**dass** von 0,008 bis 0,05 Moleq. an Katalysator bezogen auf die eingesetzte Menge an Carbonsäure eingesetzt wird.

6. Verfahren gemäß zumindest einem der Ansprüche 1 bis 5,
**dadurch gekennzeichnet,**
**dass** dem Reaktionsgemisch ein Entschäumer zugegeben wird.

7. Verfahren gemäß zumindest einem der Ansprüche 1 bis 6,
**dadurch gekennzeichnet,**
**dass** eine Carbonsäure gemäß der Formel 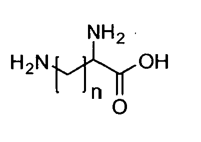 mit n = 1 bis 5 eingesetzt wird.

## Claims

1. Continuous process for decarboxylating carboxylic acids of the formula I 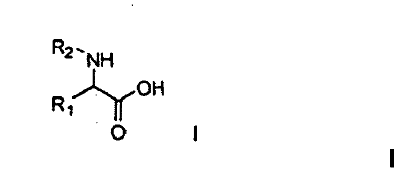 where R¹ =hydrogen, alkyl, arylalkyl, aryl, cycloalkyl or heterocycle, where the substituent of the R₁ type is substituted or unsubstituted, and
R² =hydrogen, alkyl, cycloalkyl,
**characterized in that**
I. a carbonyl compound as a catalyst is initially charged in a solvent at reaction temperature,
II. a carboxylic acid of the formula I is metered into this catalyst solution as an aqueous solution, aqueous suspension or as a water-comprising solid, and
III. a mixture of CO₂, solvent, water and the desired reaction product of the formula II and/or a salt which has an anion of the formula III 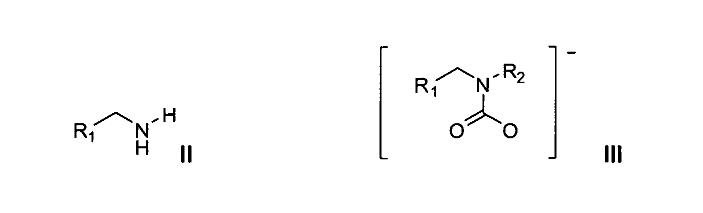 is removed continuously from the reaction mixture as a vapour.

2. Process according to Claim 1,
**characterized in that**
the solvent has a water-entraining action.

3. Process according to Claim 1 or 2,
**characterized in that**
the solvent has a boiling point of 150°C to 390°C.

4. Process according to at least one of Claims 1 to 3,
**characterized in that**
0.005 to 10 molar equivalents of catalyst based on the amount of carboxylic acid used are used.

5. Process according to Claim 4,
**characterized in that**
0.008 to 0.05 molar equivalent of catalyst based on the amount of carboxylic acid used is used.

6. Process according to at least one of Claims 1 to 5,
**characterized in that**
a defoamer is added to the reaction mixture.

7. Process according to at least one of Claims 1 to 6,
**characterized in that**
a carboxylic acid of the formula 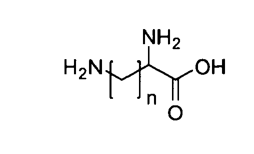 where n = 1 to 5 is used.

## Revendications

1. Procédé continu pour la décarboxylation d'acides carboxyliques selon la formule I 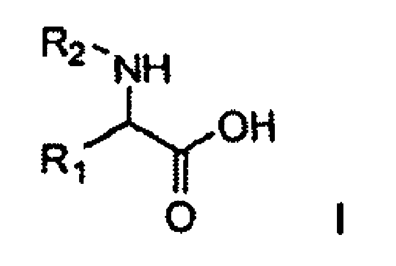
où R₁ représente un atome d'hydrogène, un groupe alkyle, arylalkyle, aryle, cycloalkyle ou un hétérocycle, le substituant du type R₁ étant substitué ou non substitué, et
R₂ représente un atome d'hydrogène ou un groupe alkyle ou cycloalkyle,
**caractérisé en ce que**
I. on dispose au préalable un composé carbonyle en tant que catalyseur dans un solvant, à la température de la réaction,
II. on ajoute par addition dosée à cette solution de catalyseur un acide carboxylique selon la formule I, sous forme de solution aqueuse, de suspension aqueuse ou de solide comportant de l'eau et
III. on évacue en continu du mélange réactionnel, sous forme de vapeur, un mélange de CO₂, solvant, eau et du produit de réaction recherché selon la formule II et/ou d'un sel qui comporte un anion selon la formule III

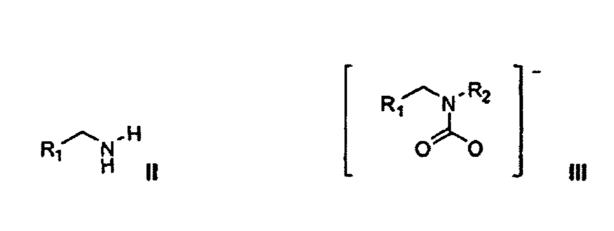

2. Procédé selon la revendication 1, **caractérisé en ce que** le solvant a un effet entraînant l'eau.

3. Procédé selon la revendication 1 ou 2, **caractérisé en ce que** le solvant a un point d'ébullition de 150 °C à 390 °C.

4. Procédé selon au moins l'une des revendications 1 à 3, **caractérisé en ce qu'**on utilise de 0,005 à 10 Eq. mol. de catalyseur, par rapport à la quantité utilisée d'acide carboxylique.

5. Procédé selon la revendication 4, **caractérisé en ce qu'**on utilise de 0,008 à 0,05 Eq. mol. de catalyseur, par rapport à la quantité utilisée d'acide carboxylique.

6. Procédé selon au moins l'une des revendications 1 à 5, **caractérisé en ce qu'**on ajoute un antimousse au mélange réactionnel.

7. Procédé selon au moins l'une des revendications 1 à 6, **caractérisé en ce qu'**on utilise un acide carboxylique selon la formule 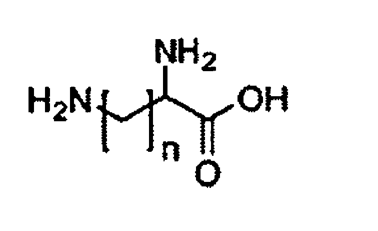 où n = 1 à 5.
